# EUROPEAN PATENT APPLICATION

(11) **EP 4 748 378 A1**
(43) Date of publication of application: **27.05.2026**
(21) Application number: 24845466.2
(22) Date of filing: 16.07.2024
(51) Int. Cl.: A61K 31/702, A61K 8/73, A61P 1/02, A61P 31/04, A61Q 11/00

(54) **PROPHYLACTIC/THERAPEUTIC AGENT FOR PERIODONTAL DISEASES**

(30) Priority: 21.07.2023 JP 2023119002
(71) Applicant: Kabushiki Kaisha Yakult Honsha, Tokyo, 105-8660 (JP)
(72) Inventor: HOKKYO Atsuko, Tokyo 105-8660 (JP); KAKIYAMA Sayaka, Tokyo 105-8660 (JP); MIZUSAWA Naomi, Tokyo 105-8660 (JP); KOBAYASHI Toshihide, Tokyo 105-8660 (JP)
(74) Representative: Wächtershäuser & Hartz Patentanwaltspartnerschaft mbB
(86) International application number: PCT/JP2024/025399
(87) International publication number: WO 2025/023085

(57) **Abstract**

A problem of the invention is to provide a prophylactic/therapeutic agent for periodontal disease which is highly safe, can be taken continuously and has an excellent prophylactic/therapeutic effect on periodontal disease. The prophylactic/therapeutic agent for periodontal disease which solves the problem of the invention is characterized by containing a galactooligosaccharide as an active ingredient.

## Description

### Technical Field

The present invention relates to a prophylactic/therapeutic agent for periodontal disease.

### Background Art

In recent years, the number of companion animals such as dogs and cats (18.5 million) greatly exceeds the population under 15 years old (about 16 million), and companion animals are regarded as family members in Japan with aging population combined with a declining birthrate. At the same time, as the average lifespan increases due to improvement of the breeding environment and improvement of the medical technology, the risk of having various diseases is also increasing.

One of such diseases is periodontal disease, and there is a report that about 80% of dogs at the age of two or older suffer from periodontal disease (NPL 1). Symptoms of periodontal disease progress with age without any appropriate care, and relation between periodontal disease and systemic diseases such as renal disease and liver disease has recently been suggested. Thus, as the lifespans of companion animals increase, prevention/improvement of periodontal disease is important for maintaining the health not only of the oral cavity but also of the whole body.

Known pathogenic bacteria of periodontal disease in companion animals include *Porphyromonas gulae* (*P. gulae*) and *Porphyromonas gingivalis* (*P. gingivalis*). *P. gulae* is a gram-negative anaerobic bacterium, and the symptoms of periodontal disease progress when biofilm is formed by *P*. *gulae.* In particular, in the case of dogs, when dental calculus deposits, subgingival anaerobic environment is maintained, and a periodontal pocket is easily formed, which then destroys the periodontal tissue and results in tooth mobility and avulsion. Moreover, it has been reported as follows: FimA, which is a main component protein of the pili that involve in attachment/colonization of *P*. *gulae* in the oral cavity, is classified into three genotypes both in dogs and cats; the detected types relate to the severity of periodontal disease; and the genotypes have a high homology to each other (NPL 2). Furthermore, it is also suggested that *P*. *gulae* spreads among humans through companion animals. It is also known that these bacteria have N-benzoyl-DL-alginyl peptidase activity. Other bacteria having the activity include *Tannerella forsythia* and *Treponema denticola.*

A general therapeutic method for periodontal disease in dogs is removal of plaque under general anesthesia, and prophylactic methods are daily tooth brushing and regular removal of plaque under anesthesia. However, daily tooth brushing is a great burden to the owners, and the removal of plaque under general anesthesia involves a risk of anesthesia and is also expensive. Therefore, various dental care products have been developed for enabling more simple oral care. For example, PTL 1 discloses a dental chew based on the growth inhibition action of probiotic bacteria such as bifidobacteria on pathogenic bacteria such as mutans streptococcus.

### Citation List

### Patent Literature

PTL 1: JP3216201U

### Non Patent Literature

NPL 1: Niemiec.B., Gawor.J., Nemec.A., et al.: World Small Animal Veterinary Association Global Dental Guidelines. J. Small Anim.Pract.,2020.61(7)
NPL 2: Naoki Iwashita, Ryota Nomura, et al.: Identification and molecular characterization of Porphyromonas gulae fimA types among cat isolates, Veterinary Microbiology, Volume 229, February 2019, Pages 100-109

### Summary of Invention

### Technical Problem

A problem of the invention is to provide a prophylactic/therapeutic agent for periodontal disease which is highly safe, can be taken continuously and has an excellent prophylactic/therapeutic effect on periodontal disease.

### Solution to Problem

As a result of extensive investigation to solve the problem, the present inventors have found that, when a galactooligosaccharide is taken, the growth or the activity of a periodontal bacterium is inhibited, and an excellent prophylactic/therapeutic effect on periodontal disease is obtained. The invention has been thus completed.

The invention is a prophylactic/therapeutic agent for periodontal disease caused by a bacterium having N-benzoyl-DL-alginyl peptidase activity containing a galactooligosaccharide as an active ingredient.

Moreover, the invention is a prophylactic/therapeutic agent for periodontal disease for a companion animal or an inhibitor of a periodontal bacterium for a companion animal containing a galactooligosaccharide as an active ingredient.

Furthermore, the invention is a method for preventing/treating periodontal disease of a companion animal characterized by administering a galactooligosaccharide to a companion animal.

### Advantageous Effects of Invention

The prophylactic/therapeutic agent for periodontal disease of the invention is highly safe, can be taken daily and can prevent/treat periodontal disease simply and effectively by inhibiting the growth or the activity of a periodontal bacterium.

### Brief Description of Drawings

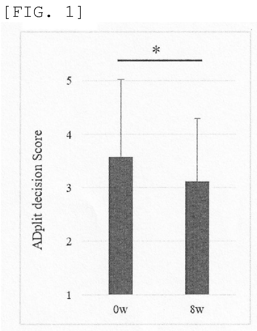

[FIG. 1] The N-benzoyl-DL-alginyl peptidase activities (the periodontal bacterial levels (average values ± standard deviations, n=26, *: p < 0.05)) of the plaque samples of dogs before the galactooligosaccharide intake and after eight weeks of intake in Example 1.

### Description of Embodiments

The prophylactic/therapeutic agent for periodontal disease of the invention contains a galactooligosaccharide as an active ingredient. Galactooligosaccharide is a generic term for oligosaccharides having at least one or more galactose residues in the molecule, and examples thereof include saccharides in which two to nine, preferably three or four monosaccharides are linked. Moreover, the galactooligosaccharide may be a galactooligosaccharide in which galactose is linked by a β1-2 bond, a β1-3 bond, a β1-4 bond or a β1-6 bond, but a galactooligosaccharide having a β1-4 bond or a β1-6 bond is particularly preferable.

More preferable galactooligosaccharides include β-1,4 galactosyllactose (Galβ1-4Galβ1-4Glc), β-1,6 galactosyllactose (Galβ1-6Galβ1-4Glc), β-1,3 galactosyllactose (Galβ1-3Galβ1-4Glc) and the like, and in view of the prophylactic/therapeutic effect on periodontal disease, a galactooligosaccharide containing β-1,4 galactosyllactose as a main component is more preferable. For example, OLIGOMATE 55N (manufactured by Yakult Pharmaceutical Industry Co., Ltd., the galactooligosaccharide content of the solid contents is 55 mass%, and the β-1,4 galactosyllactose content is 18.5 mass%) can be used.

The prophylactic/therapeutic agent for periodontal disease of the invention can achieve a prophylactic/therapeutic effect on periodontal disease by inhibiting the growth or the activity of a periodontal bacterium in the oral cavity. In the invention, periodontal disease is an infection of the periodontal tissue with a periodontal bacterium and includes gingivitis, periodontitis, alveolar pyorrhea and the like. The periodontal bacterium is a bacterium having N-benzoyl-DL-alginyl peptidase activity, and specific examples thereof include *Porphyromonas gulae, Porphyromonas gingivalis, Tannerella forsythia, Treponema denticola* and the like. The prophylactic/therapeutic agent for periodontal disease of the invention can be applied to a human and a non-human mammal, and examples of the non-human mammal include companion animals such as dogs, cats, rabbits and hamsters and domestic animals such as pigs and cows. Of these, a companion animal is preferable, and a dog or a cat is further preferable. The agent is particularly preferably used for a dog.

The form of the prophylactic/therapeutic agent for periodontal disease of the invention is not particularly restricted, and the form can be a medicine, a food or a drink or a supplement for a human, a pet food, a supplement for a pet, a pharmaceutical product for a pet or the like. The pharmaceutical product can be formulated from a galactooligosaccharide as it is or in combination with a pharmaceutically acceptable carrier according to the need. Examples of the pharmaceutically acceptable carrier include glucose, lactose, starch, mannitol, dextrin, fatty acid glycerides, polyethylene glycol, hydroxyethyl starch, ethylene glycol, polyoxyethylene sorbitan fatty acid esters, amino acids, gelatine, albumin, water, physiological saline and the like. Furthermore, according to the need, a generally used additive such as a stabilizer, a moisturizer, an emulsifier, a binder, an isotonic agent and an excipient can also be appropriately added. The dosage form thereof is not particularly limited, but examples thereof include a liquid agent, a powder agent, granules, capsules, tablets and the like which can be produced according to a normal method. The galactooligosaccharide content of the prophylactic/therapeutic agent for periodontal disease of the invention is not particularly restricted and is, for example, preferably 1 to 100 mass%, more preferably 5 to 70 mass%, further preferably 10 to 60 mass%.

The dose of the prophylactic/therapeutic agent for periodontal disease or the like of the invention is not particularly restricted, but for example, a daily dose for a human adult per 1 kg body weight is around 0.03 to 0.4 g, preferably around 0.04 to 0.3 g, particularly preferably around 0.1 to 0.25 g. The dose for the case of a non-human mammal such as a companion animal can be the same dose.

In the case of a form of a food or a drink, the food or the drink is prepared according to a normal method from a galactooligosaccharide as it is or by blending a known food additive and/or food material according to the need. The form thereof is not particularly restricted, and examples thereof include foods mainly containing starch such as bread, biscuits, pancakes, noodles and tablet candies, confectionery such as gums, candies and Japanese confectionery, livestock products such as hams and sausages, fish products such as *chikuwa* (hollow cylindrical fish sausages) and *kamaboko* (steamed fish paste), fish and shellfish products, seasonings such as dressing, soy sauce, jam and *furikake* (dry Japanese seasonings), drinks such as tea, juice, soft drinks and alcohols and the like.

In the case of a form of a pet food or a supplement for a pet, the pet food or the supplement for a pet can be produced from a galactooligosaccharide as it is or by appropriately mixing a raw material used for a known pet food or a known supplement for a pet according to the need. Examples of such a raw material include meat such as beef, chicken and pork, fish and shellfish such as bonito, mackerel, scallop and short-necked clam, grains such as corn, barley, wheat and rye, starch such as corn starch, wheat starch and rice starch, dairy products such as butter and cheese, vitamins such as vitamins A, B1, B2, D and E, niacin and pantothenic acid, minerals such as iron, calcium, sodium and potassium, probiotics and the like. Moreover, an additive such as a gelling agent, a shape-retaining agent, a pH-adjusting agent, a seasoning, a preservative and a nutrition-reinforcing agent may also be blended.

The pet food or the supplement for a pet can be obtained by mixing the components according to a normal method and forming into any form such as a dry type, a wet type, jerky, chew, particles, powder and a drink.

### Examples

The invention is explained in detail below referring to Examples of the invention, but the invention is not limited to the Examples.

### Example 1

Dogs were caused to take galactooligosaccharides (GOSs), and the effect on periodontal bacteria was examined. Twelve small-size dogs (less than 5 kg), nine middle-size dogs (5 to 10 kg) and five large dogs (exceeding 10 kg) which were kept at home, namely 26 dogs in total, were used as subjects. The female:male ratio was 9:17, the average age was 8.88 years old, and the average body weight was 7.76 kg. The doses were set as shown in Table 1, and OLIGOMATE 55N (liquid galactooligosaccharides; Yakult Pharmaceutical Industry Co., Ltd.) was fed for eight weeks. The feeding was either by a method of putting the predetermined amount of OLIGOMATE 55N on a spoon and allowing direct oral intake or by a method of giving by pouring on feed. The statistical analysis was conducted by Wilcoxon signed-rank sum test. It was determined that there was a significance difference in the case of p < 0.05.

**[Table 1]**

| Body Weight | 5 kg< | 5 to 10 kg | <10 kg |
|---|---|---|---|
| OLIGOMATE 55N (GOS content) | 1.2 g (0.5 g) | 2.4 g (1.0 g) | 4.8 g (2.0 g) |

Plaque samples were taken from the surface close to the gingiva of a maxillary canine or molar tooth of the dogs before the galactooligosaccharide intake and after the eight-week intake. Then, using an activity assay kit (ADplit (registered trademark); Kyoritsu Seiyaku Corporation) for N-benzoyl-DL-alginyl peptidase, which is an enzyme specific to *Porphyromonas gulae* and the like, according to the descriptions in the attached document, the N-benzoyl-DL-alginyl peptidase activities were measured, and the periodontal bacterial levels were evaluated by five-point scale. A higher value thereof indicates higher enzymatic activity. The results are shown in FIG. 1. The score after the eight-week galactooligosaccharide intake (3.12) decreased with significance compared to that before the intake (3.58), and it was found that galactooligosaccharides have an action of inhibiting periodontal bacteria.

Moreover, blood samples were taken before the galactooligosaccharide intake, after four weeks and eight weeks of the intake and four weeks after the completion of the intake, and the biochemical blood data, such as total protein, albumin, total bilirubin, blood urea nitrogen and creatinine, were measured. Because no change in the data which was believed to be an adverse event was observed, it was found that there was no problem with the safety.

### Industrial Applicability

The prophylactic/therapeutic agent for periodontal disease of the invention is highly safe, can be taken daily and continuously for a long time and exerts an excellent prophylactic/therapeutic effect on periodontal disease of a dog, a cat or the like, and thus the prophylactic/therapeutic agent can be used as a pet food, a supplement for a pet or the like used for periodontal disease.

## Claims

1. A prophylactic/therapeutic agent for periodontal disease caused by a bacterium having N-benzoyl-DL-alginyl peptidase activity, comprising a galactooligosaccharide as an active ingredient.

2. The prophylactic/therapeutic agent for periodontal disease according to claim 1, wherein the bacterium having N-benzoyl-DL-alginyl peptidase activity is *Porphyromanas gulae* or *Porphyromonas gingivalis.*

3. A prophylactic/therapeutic agent for periodontal disease for a companion animal, comprising a galactooligosaccharide as an active ingredient.

4. An inhibitor of a periodontal bacterium for a companion animal, comprising a galactooligosaccharide as an active ingredient.

5. A method for preventing/treating periodontal disease of a companion animal **characterized by** administering a galactooligosaccharide to a companion animal.
